# EUROPEAN PATENT SPECIFICATION

(11) **EP 2 322 158 B1**
(45) Date of publication and mention of the grant of the patent: **10.06.2020**
(21) Application number: 11150018.7
(22) Date of filing: 25.10.2006
(51) Int. Cl.: A23L 2/52, A23L 33/105, A61K 36/87, A61K 31/05, A61K 47/22, A61K 9/00, A61K 9/20, A61K 9/48

(54) **RESVERATROL AND/OR GRAPE LEAF EXTRACT FOR ENERGY METABOLISM ACTIVATION**
RESVERATROL UND/ODER TRAUBENBLATTEXTRAKT ZUR AKTIVIERUNG DES ENERGIEMETABOLISMUS
RESVERATROL ET/OU EXTRAIT DE GRAPPES POUR ACTIVIER LE METABOLISME ENERGETIQUE

(30) Priority: 26.10.2005 JP 2005311504
(43) Date of publication of application: 18.05.2011
(62) Divisional of application: 06822740.4
(73) Proprietor: KAO CORPORATION, Tokyo 103-8210 (JP)
(72) Inventor: Minegishi, Yoshihiko, Tochigi 321-3497 (JP); Murase, Takatoshi, Tochigi 321-3497 (JP); Haramizu, Satoshi, Tochigi 321-3497 (JP)
(74) Representative: Hoffmann Eitle

(56) References cited:
- WO-A1-00/21526
- WO-A1-2005/028464
- WO-A2-2005/069998
- WO-A2-2007/008548
- YANG YING-BAO ET AL: "Effects of resveratrol on secondary damages after acute spinal cord injury in rats.", ACTA PHARMACOLOGICA SINICA, vol. 24, no. 7, July 2003 (2003-07), pages 703-710, XP009077328, ISSN: 1671-4083
- SINGH AMANPREET ET AL: "Effect of natural and synthetic antioxidants in a mouse model of chronic fatigue syndrome.", JOURNAL OF MEDICINAL FOOD, vol. 5, no. 4, January 2002 (2002-01), pages 211-220, XP002654505, ISSN: 1096-620X
- LOGAN A C ET AL: "Chronic fatigue syndrome: oxidative stress and dietary modifications", ALTERNATIVE MEDICINE REVIEW, vol. 6, no. 5, 1 October 2001 (2001-10-01) , pages 450-459, XP009150507, THORNE RESEARCH INC., SANDPOINT, US ISSN: 1089-5159

## Description

### Technical Field to which the Invention Belongs

The present invention relates to the non-therapeutic use of resveratrol for improving endurance in exercise, for anti-fatigue in exercise and for improving aging-induced reduction of energy metabolism in a human or animal.

In recent years, owing to development of transportation means and progress of information and communication technology, people have been short of exercise. Insufficient exercise leads to decrease in motor function such as decrease in endurance or muscle strength and is presumed to have a serious negative influence on their future QOL.

Aging-induced muscle atrophy or decrease in endurance or muscle strength leads to deterioration in motor function, which may sometimes confine the elderly to bed. In addition, bone fracture followed by falling during daily performance owing to muscle atrophy or lowering in muscle power is the major cause of the elderly becoming bedridden.

It is therefore important to usually maintain or enhance the motor function and prevent aging-induced reduction in the motor function in order to maintain and improve the daily life function and extend healthy life expectancy.

Exercise training is suited for improving the motor function, but there are many people who are now too busy to take sufficient exercise or who get tired easily during exercise and cannot continue the exercise. The elderly are recommended to take moderate exercise or continue physical rehabilitation in order to prevent reduction in motor performance, but it is difficult to do so in daily life because of decline in their motivation or risk of injury. There is accordingly a demand for the development of more effective method.

One of the measures for overcoming these problems is daily intake of a food component having a motor function enhancing effect such as an endurance improving or muscle force improving or having an anti-fatigue effect. For example, the present inventors have found that intake of catechins is effective for improving endurance (JP-A-2005-89384). Additional reported examples of the component having an endurance improving effect include a hawthorn extract (JP-A-8-47381), a Reishi mushroom component (JP-A-5-123135), and proanthocyanidin and lycopene (JP-A-2005-334022). Examples of a component having an anti-fatigue effect include a mixture of coenzyme Q10 and carnitine (JP-A-2005-97161) and glutamine peptide (JP-A-200S-97162) and so on.

As a component capable of regulating motor performance, disclosed are, for example, an anti-aging agent containing cystine and theanine (WO2005-123058), a muscle atrophy inhibitor containing a fruit polyphenol (JP-A-2001-89387), a muscle protein degradation inhibitor containing lycopene (JP-A-2004-59518), a motor performance deterioration inhibitor having as an effective ingredient an organic acid having at least two carboxyl groups or salts thereof (JP-A-10-17585), an anti-fatigue agent containing astaxanthin and/or ester thereof (JP-A-2006-16409), and a muscle atrophy inhibitor and motor performance improver containing proanthocyanidin (JP-A-2002-338464, JP-A-2005-97273). However, their practical inhibitory effects on deterioration in endurance, muscle strength and energy metabolism and on muscle atrophy, each induced by aging, have not yet been studied.

Resveratrol is a polyphenol contained in grapes, wines made therefrom, and peanuts and it has been taken for long years. Resveratrol is reported to have an anti-cancer effect (Science, 275(5297), 218-220(1998)) or an antiatherogenic effect (Cardiovasc. Drug Rev., 22(3), 169-188(2004)). It is also reported to have a life- extending effect mediated by a molecule called Sir (Nature, 425(6954), 191-196(2003)). In addition, it is disclosed that resveratrol is useful for the prevention or treatment of hyperlipidemia (JP-A-2001-72853) or prevention or treatment of cerebral/aging-induced diseases (JP-A-2002-527389).

On the other hand, a red grape leaf extract has traditionally been known as a therapeutic agent for venous incompetence and patents on a blood circulation improving method by a red grape leaf extract (JP-A-2006-516542) and a therapy for chronic venous incompetence (JP-A-2003-511476) are disclosed.

However, influences of resveratrol or grape leaf extract on motor performance including endurance and fatigue, and influences on aging-induced reduction in endurance and energy metabolism have been hitherto unknown. Yang, Ying-Bao et al., Acta Pharmacologica Sinica, vol. 24, no. 7, July 2003 (2003-07), pages 703-710, XP009077328, ISSN: 1671-4083 describes the effects of resveratrol on secondary damages after acute spinal cord injury in rats. The impact of resveratrol on secondary spinal cord edema, lactate dehydrogenase (LDH) activity, Na/K-ATPase activity Malondialdehyde (MDA) formation are examined. Yang, Ying-Bao et al., Acta Pharmacologica Sinica, vol. 24, no. 7, July 2003 (2003-07), pages 703-710, XP009077328, ISSN: 1671-4083 describes the effects of resveratrol (Res) on secondary damages after acute spinal cord injury in rats. The impact of resveratrol on secondary spinal cord edema, lactate dehydrogenase (LDH) activity, Na/K-ATPase activity Malondialdehyde (MDA) formation are examined.

WO 2005/028464 A1 concerns an endurance improving agent or anti-fatigue agent which containing catechin as an active ingredient.

Singh Amanpreet et al., Journal of Medicinal Food 5, 2002, 211-220 describe the effect of natural and synthetic antioxidants on chronic fatigue syndrome (CFS). It states that antioxidants may be beneficial in the management of CFS and that antioxidants such as carvedilol, melatonin, St. John's wort, W. somnifera, and quercetin could be used as potential agents in the management of CFS.

According to the Search Opinion, the teaching of D3 relating to quercetin is deemed particularly important. Quercetin is a flavonoid, however, whereas resveratrol is a stilbenoid (see paragraph 14 of the present application).

Logan, A. C. et al., Alternative Medicine Review, vol. 6, no. 5, 1 October 2001 (2001-10-01), pages 450-459 teach that antioxidants such as oligomeric proanthocyanidins may be helpful against CFS.

### SUMMARY OF THE INVENTION

WO 2005/028464 A1 concerns an endurance improving agent or anti-fatigue agent which containing catechin as an active ingredient.

Singh Amanpreet et al., Journal of Medicinal Food 5, 2002, 211-220 describe the effect of natural and synthetic antioxidants on chronic fatigue syndrome (CFS). It states that antioxidants may be beneficial in the management of CFS and that antioxidants such as carvedilol, melatonin, St. John's wort, W. somnifera, and quercetin could be used as potential agents in the management of CFS.

According to the Search Opinion, the teaching of D3 relating to quercetin is deemed particularly important. Quercetin is a flavonoid, however, whereas resveratrol is a stilbenoid (see paragraph 14 of the present application).

Logan, A. C. et al., Alternative Medicine Review, vol. 6, no. 5, 1 October 2001 (2001-10-01), pages 450-459 teach that antioxidants such as oligomeric proanthocyanidins may be helpful against CFS.

WO 2007/008548 is a post-published document relating to methods and compositions for treating or preventing metabolic disorders, such as obesity and diabetes. The methods may comprise modulating the activity or level of a sirtuin, such as SIRTI or Sir2. Exemplary methods comprise contacting a cell with a sirtuin activating compound, such as a flavone, stilbene, flavanone, isoflavone, catechin, chalcone, tannin or anthocyanidin, or an inhibitory compound, such as nicotinamide.

WO 2005/069998 concerns methods for preparing resveratrol, resveratrol esters and substituted and unsubstituted stilbenes of a certain formula. These compounds show increased stability for use in the food, cosmetic and pharmaceutical industries.

WO 00/21526 describes a pharmaceutical product which comprises admixed or separately packaged (A) L-carnitine or an alkanoyl L-carnitine (e.g.: acetyl L-carnitine or propionyl L-carnitine) and (B) a trihydroxy or tetrahydroxystilbene (e.g.: resveratrol). The product is said to be useful for the prevention and treatment of pathological forms due to neuronal or cerebral disorders.

### SUMMARY OF THE INVENTION

The present invention relates to the following items (1) to (3):
(1) Non-therapeutic use of resveratrol for improving aging-induced reduction of energy metabolism in a human or animal.
(2) Non-therapeutic use of resveratrol for improving endurance in exercise in a human or animal.
(3) Non-therapeutic use of resveratrol for anti-fatigue in exercise in a human or animal.

### DETAILED DESCRIPTION OF THE INVENTION

The present invention relates to the non-therapeutic use of resveratrol for improving endurance in exercise, for anti-fatigue in exercise and for improving aging-induced reduction of energy metabolism in a human or animal.

The present inventors have searched for components effective for improving the body function. As a result, they have found that among natural materials which have been taken as a food for long years and have high safety, resveratrol has an endurance improving action, anti-fatigue action, and action of inhibiting aging-induced reduction in energy metabolism, and therefore is useful as an endurance improver, anti-fatigue agent or anti-aging agent for inhibiting aging-induced reduction in energy metabolism.

Resveratrol according to the present invention is useful as a food or drug exhibiting an endurance improving effect in sports and also broadly-defined exercises including activities of daily life and labors and an anti-fatigue effect. It is also useful as a food or drug having effects of inhibiting aging-induced reduction in energy metabolism. These uses employ resveratrol as an effective ingredient which has been taken for long years as a food, and have high safety with fewer side effects.

The term "resveratrol" as used herein means 3, 4',5-trihydroxy-stilbene and it embraces trans-resveratrol and cis-resveratrol, and a mixture thereof. In the present invention, at least one of them is used. Resveratrol of the present invention may be a glycoside (piceid or the like).

The resveratrol used for the present invention may be not only a high purity product synthesized in accordance with organic chemistry or by using a microorganism but also products obtained by extracting resveratrol-containing natural products such as grapes, peanuts, plants belonging to the family *polygonaceae* such as Japanese knotweed and then purifying the extract as needed. And Plants, microorganisms, or extracts, partially purified products or processed products of various foods may also be used. Resveratrol isolated from these extracts, partially purified products or processed products may also be used.

As the natural products, grapes and Japanese knotweed are preferred. Grapes can be classified into European grapes (*Vitis vinifera*) and American grapes (*Vitis labrusca*). Either can be used, but European grapes are preferred. Examples of the grapes include Delaware, Kyoho, Koshu, Pione, Muscat, Chenin Blanc, Grenache, Mataro, Muller Thurgau, Trebbiano, Berry A, Cabernet Sauvignon, Merlot, Pinot Noir, Cabernet Franc, Syrah, Chardonnay, Sauvignon Blanc, Semillon, Syrah, Gamay, Riesling, Aligote, Muscadelle, Sauvignon, Folle Blanche, Muscadet, Chenin, Grolleau, Pineau d'Aunis, Pinot Meunier, Gewurztraminar, Pinot Grigio, Sylvaner, Poulsard, Savagnin, Jacquere, Mondeuse, Roussette, Carignan, Cinsaut, Clairette, Grenache Noir, Mourvedre, Ugni Blanc, Nielluccio, Sciacarello, Vermentino, Marsanne, Roussanne, Viognier, Carignan, Cinsaut, Clairette, Mourvedre, Bourboulenc, Carignan, Cinsaut, Maccabeu, Mourvedre, Mauzac, Tannat, Colombard, Len De Lel, Malbec, Petit Manseng, Gros Manseng, Petit Verdot, Sangiovese, Nebbiolo, Barbera, Dolcetto, Carinena, Garnacha, Monastrell, and Tempranillo;

Examples of the grape extract include extracts available from grape berries, grape leaves and products derived from grapes. Of these, a grape leaf extract, especially a red grape leaf extract is preferred. A red grape leaf extract from European grapes (*Foliae Vitis vinifera L*.) is especially preferred. Examples of the products derived from grapes include grape juice, wine, residues and juice which are by-products upon wine production, and wine concentrate.

Since Resveratrol is contained much in skins or leaves of grapes, an ordinary extraction process of extracting them with water, hot water, aqueous alcohol, organic solvent or the like can be adopted for extraction.

In addition, Resveratrol is also contained in grape juice, wine, concentrated juice or wine, or solid product obtained by concentrating them to dryness, so they can be provided for use. In this case, the residue of grapes which has appeared when they are produced may be used as they are or resveratrol can be obtained by extracting the residue with an organic solvent/water or the like.

As the organic solvent to be used for the extraction of resveratrol, alcohols such as ethanol are preferred, and ethanol is especially preferred.

A Japanese knotweed contains much resveratrol in its root, so Japanese knotweed extract obtained by subjecting this portion to an ordinary extraction process similar to the above-described one is preferred.

The resveratrol used in the present invention has, as described later in Examples, an endurance improving action, anti-fatigue action, and an anti-aging action, more specifically, an action of inhibiting aging-induced reduction in energy metabolism. The resveratrol can therefore be used as an endurance improver, anti-fatigue agent, and energy metabolism activator for inhibiting aging-induced reduction of energy metabolism (which will hereinafter be called "endurance improver and the like") and moreover can be used for the preparation of these agents. The endurance improver and the like are usable as a human or animal drug, quasi drug or food which is effective for improving endurance, preventing fatigue, and anti-aging. The resveratrol and/or grape leaf extract can also be used for a food, functional food, invalid diet or food for specified health use which is produced with a view to improving endurance, suppressing fatigue, or inhibiting aging-induced reduction of energy metabolism. Moreover, it can be used not only for those who are short of exercise, middle and aged persons or those needing bed rest, but also for all people including athletes.

The term "anti-aging" as used herein means suppression of an aging-induced reduction of energy metabolism.

When the endurance improver and the like according to the present invention are employed as a drug or quasi drug, their dosage forms include orally administrable ones such as tablets, capsules, granules, powders and syrups, and parenterally administration ones such as injections, suppositories, inhalation drugs, percutaneous absorbents, and external preparations. Preparations in such various dosage forms can be prepared-using the resveratrol and/or grape leaf extract of the present invention singly or in combination with pharmaceutically acceptable additives such as excipient, binder, extender, disintegrator, surfactant, lubricant, dispersant, buffer, preservative, taste corrigent, flavor, film forming agent, carrier and diluent as needed. Of these dosage forms, oral administration is preferred. An orally administrable liquid preparation can be prepared in a manner known *per se* in the art by the addition of a taste corrigent, buffer, stabilizer and the like.

When the energy metabolism activator according to the present invention is used as a food, it can be used in the forms of various foods such as breads, cakes, noodles, confectioneries, jellies, frozen foods, ice creams, milk products, beverages and soups and also in the forms similar to those described above in orally administrable preparations (tablets, capsules, syrups and the like). The beverages include fruit juices, carbonated beverages, tea beverages, near water drinks, sports drinks, milk beverages, alcoholic beverages and soft drinks.

When foods of various forms are prepared, the resveratrol used in the present invention may be used either singly or in combination with another food material or solvent, softener, oil, emulsifier, antiseptic, flavor, stabilizer, colorant, antioxidant, humectant and/or thickener as needed. The foods thus prepared can be used as an endurance improving food, muscle strength improving food, motor function improving food, anti-fatigue food or anti-aging food or pet food having such a purpose.

The energy metabolism activator according to the present invention can be used as a nutritional composition such as enteral nutrient for the elderly or patients needing bed rest who need feeding of an adequate amount of nutrients;

The amount of the resveratrol to foods, quast drugs and the like differs, depending on their form of use. To foods, resveratrol is added usually in an amount of from 0.0001 to 5 mass%, preferably from 0.001 to 2 mass%, more preferably from 0.002 to 1 mass%. To oral solid preparations such as tablets, granules and capsules and oral liquid preparations such as internal solutions and syrups, resveratrol is added usually in an amount of from 0.01 to 95 mass%, preferably from 5 to 90 mass%, more preferably from 10 to 50 mass%.

The daily dose (effective intake) of the resveratrol according to the present invention is set preferably at from 1 to 2000 mg/60 kg-body weight. More specifically, the daily dose of resveratrol is set preferably at from 3 to 700 mg/60 kg-body weight, more preferably from 5 to 500 mg/60 kg-body weight, even more preferably from 20 to 400 mg/60 kg-body weight.

Tests and Examples which are typical in the present invention will next be described.

### Examples

### Test 1 (endurance improving · anti-fatigue effect of resveratrol)

The endurance-improving/anti-fatigue effect of resveratrol were evaluated in the below-described manner. As resveratrol, that available from Cayman Chemical was employed.

An endurance-improving/anti-fatigue effect was evaluated in accordance with the mice maximal swimming time measurement (Reference: Am. J. Physiol. Regul. Integr. Comp. Physiol., 288, 708-715(2005)) in an adjustable-current swimming pool. The pool used for this test made of transparent acryl (90 cm long × 45 cm wide × 45 cm deep) was filled with water to the depth of 38 cm and maintained at 34°C by a heater. The current in the pool was generated by circulating water with a pump ("C-P60H", product of Hitachi, Ltd.). The flow rate was regulated by opening and closing of a valve of a water flow meter (product of Tokyo Flow Meter Kenkyujo) connected to the pump. The flow rate was monitored by a current meter ("SV-101-25S", product of Sanko Seimitsu Kogyo).

As a test animal, five-week-old Balb/c male mice (Charles River) were used. They were habituated by preliminary rearing for 1 week under a predetermined rearing environment (23 ± 2°C, light period: from 7:00 am to 7:00 pm). For the next one week, training was performed three times a week in order to accustom the mice to swimming (swimming for 30 minutes at a flow rate of 5 L/min on Day 1, and for 30 minutes at a flow rate of 6 L/min on Day 3 and Day 5). For the next one week, after the mice were fasted for 2 hours, their maximal swimming time (the time point when each mouse was rescued because it failed to come up to the surface of water for breathing was defined as the maximal swimming time) at a flow rate of 7 L/min was measured twice. To prevent a difference between groups in the maximal swimming time, mice were classified into two groups each consisting of 8 mice, that is, a test diet group and control diet group. They were reared for 10 weeks on diets prepared according to the formulation as shown in Table 1. During this term, maximal swimming time measurement (7 L/min) and training (6 L/min, 30 minutes) were each conducted once a week. The maximal swimming time of mice on Week Ten after the rearing was started is shown in Table 2.

**Table 1**

| Diet Composition (mass%) | | |
|---|---|---|
| | Control diet | Test diet |
| Lipid (%) | 10 | 10 |
| Casein (%) | 20 | 20 |
| Potato starch (%) | 55.5 | 55.3 |
| Cellulose (%) | 8.1 | 8.1 |
| Vitamin (%) | 2.2 | 2.2 |
| Methionine (%) | 0.2 | 0.2 |
| Mineral (%) | 4 | 4 |
| Resveratrol (%) | 0 | 0.2 |
| Total (%) | 100 | 100 |

**Table 2**

| Maximal swimming time before rearing and after rearing for 10 weeks. | | | | |
|---|---|---|---|---|
| | Before rearing | | After rearing for 10 weeks | |
| | Maximal swimming time (min) | Statistical significance | Maximal swimming time (min) | Statistical significance |
| Control diet | 28.40±1.08 | / | 31.15±2.57 | / |
| Test diet | 28.50±1.75 | N.S | 44.80±4.56 | p < 0,05 |

| | | | | |
|---|---|---|---|---|
| * Statistical significance is relative to the control group (t-test) | | | | |

The results in Table 2 reveal that compared with the mice of the control diet group, the mice of the resveratrol-containing test diet group significantly extended the maximal swimming time after rearing for 10 weeks.Therefore resveratrol has an endurance improving effect and an anti-fatigue effect; and that it has a motor function improving effect. For this reason Resveratrol is useful as an endurance improver, anti-fatigue agent and motor function improver.

### Test 2: Endurance-improving/anti-fatigue effect of grape leaf extract

The endurance improving/anti-fatigue effect of the grape leaf extract and the grape seed extract were evaluated in the following manner. As the grape leaf extract, a red grape leaf dry extract of ASK INTERCITY was used. As the grape seed extract, Grape Seed Extract (95%) of Organic Herb was used.

Test 2 was performed in a similar manner to Test 1 except for the use of a diet prepared according to the formulation as shown in Table 3. The maximal swimming time of mice on Week Ten after rearing is shown in Table 4.

**Table 3**

| Diet Composition (mass%) | | | |
|---|---|---|---|
| | Control diet | Grape leaf extract diet | Grape seed extract diet |
| Lipid (%) | 10 | 10 | 10 |
| Casein (%) | 20 | 20 | 20 |
| Potato starch (%) | 55.5 | 55.0 | 55.0 |
| Cellulose (%) | 8.1 | 8.1 | 8.1 |
| Vitamin (%) | 2.2 | 2.2 | 2.2 |
| Methionine (%) | 0.2 | 0.2 | 0.2 |
| Mineral (%) | 4 | 4 | 4 |
| Red grape leaf dry extract (%) | 0 | 0.5 | 0 |
| Grape seed extract (%) | 0 | 0 | 0.5 |
| Total (%) | 100 | 100 | 100 |

**Table 4**

| Maximal swimming time before rearing and after rearing for 10 weeks | | | | |
|---|---|---|---|---|
| | Before rearing | | After rearing for 10 weeks | |
| | Maximal swimming time (min) | Statistical significance | Maximal swimming time (min) | Statistical significance |
| Control diet | 25.52±1.76 | / | 32.27±1.40 | / |
| Grape leaf extract diet | 25.48±1.46 | N.S | 44.28±4.40 | p < 0.05 |
| Grape seed extract diet | 25.49±1.63 | N.S | 37.13±3.29 | N.S |

| | | | | |
|---|---|---|---|---|
| * Statistical significance is relative to the control group (t-test) | | | | |

The results in Table 4 have revealed that compared with the mice of the control diet group, the mice of the red grape leaf extract-containing test diet group significantly extended the maximal swimming time after rearing for 10 weeks and therefore, the grape leaf extract has an endurance improving effect and anti-fatigue effect; and that it has a motor function improving effect. On the other hand, the mice of the grape seed extract-containing test diet group also showed extension of the maximal swimming time compared to the mice of the control diet group, but the degree of the extension is lower than that in the mice of the grape leaf extract group, and the extension is not a significant level. The grape leaf extract and grape seed extract both contain polyphenols, anthocyanins etc., however, the test results revealed that the composition of the components contained in the grape leaf extract is more effective as endurance improving agent, anti-fatigue agent, and motor function improving agent.

The grape leaf extract is therefore useful as an endurance improver, anti-fatigue agent and motor function improving agent.

### Test 3: Anti-aging effect of resveratrol

An anti-aging effect of resveratrol was evaluated in the below-described manner. Resveratrol available from Organic Herb Inc was used.

After preliminary individual rearing of 13-week-old SAM-P1 male mice (senescence accelerated mice) and SAM-R1 male mice (ordinarily aged mice) for 5 weeks, they were habituated to treadmill running and their maximal running time was measured. The measurement was started after the mice were kept sedentary in the treadmill to accustom them to the environment. The belt speed was started at 10 m/min. The mice were caused to run for 5 minutes on the belt having a speed of 10 m/min, 5 minutes at a speed of 15 m/min, 60 minutes at a speed of 20 minutes, 60 minutes at a speed of 22 m/min, 60 minutes at a speed of 24 m/min, 60 minutes at a speed of 26 m/min and then at a speed of 28 m/min. The time point when the mouse failed to run on the treadmill was determined as the maximal running time and their endurance in exercise was defined. To avoid a difference in the maximal running time between strains, the mice were divided into three groups, that is, SAM-R1 control group, SAM-P1 control group, and SAM-P1 resveratrol group (each group consisting of 5 to 8 mice).

They were reared for 10 weeks by using a diet prepared according to the formulation as shown in Table 5. Each mouse was exercised at 15 m/min for 30 minutes three times a week during the period of experiment.

**Table 5**

| Feed Composition (wt%) | | |
|---|---|---|
| | Control group | Resveratrol group |
| Casein | 20% | 20% |
| DL-Methionine | 0.2% | 0.2% |
| Lipid | 10% | 10% |
| Potato starch | 55.5% | 55.3% |
| Cellulose | 8.1% | 8.1% |
| Mineral | 4% | 4% |
| Vitamin | 2.2% | 2.2% |
| Resveratrol | 0% | 0.2% |
| Total | 100% | 100% |

The maximal running time of each group was measured after rearing for 8 weeks. The maximal running time of mice at this time is shown in Table 6. In addition, as energy metabolism at rest, oxygen consumption of each group was measured by breath analysis after rearing for 9 weeks. For this analysis, "Oxymax system" (product of Columbus Instruments) equipped with 8 chambers was used. After a mouse was placed in the chamber and kept sedentary for 6 hours, measurement was made for two straight minutes every 18 minutes for 24 hours. The average oxygen consumption for 24 hours is shown in Table 7. On the final day of the experiment, the mice were sacrificed and gastrocnemius muscle, soleus muscle and plantaris muscle were dissected. From their weights, total muscle weight was determined. The total muscle weight per body weight is shown in Table 8.

**Table 6**

| Maximal running time of mice after rearing for 8 weeks | | |
|---|---|---|
| | After rearing for 8 weeks | |
| | Maximal running time (min) | Statistical significance |
| SAM-R1 control group | 154.8 ± 93 | *p<0.05 |
| SAM-P1 control group | 100.0 ± 5.8 | / |
| SAM-P1 Resveratrol group | 141.3 ± 19,7 | *p<0.05 |

| | | |
|---|---|---|
| Statistical significance relative to SAM-P1 control group (Fisher test) | | |

**Table 7**

| Average oxygen consumption for 24 hours after rearing for 9 weeks | | |
|---|---|---|
| | After rearing for 9 weeks | |
| | Oxygen consumption (mL/kg/min) | Statistical significance |
| SAM-R1 control group | 53.2 ± 1.8 | *p<0.05 |
| SAM-P1 control group | 46.9 ± 1.3 | / |
| SAM-P1 Resveratrol group | 57.1 ± 4.3 | *p<0.05 |

| | | |
|---|---|---|
| Statistical significance relative to SAM-P1 control group (Fisher test) | | |

**Table 8**

| Muscle weight per body weight after rearing for 10 weeks | | |
|---|---|---|
| | After rearing for 10 weeks | |
| | Relative muscle weight (%) | Statistical significance |
| SAM-R1 control group | 0.93 ± 0.03 | *p<0,05 |
| SAM-P1 control group | 0.64 ± 0,02 | / |
| SAM-P1 Resveratrol group | 0.81 ± 0.07 | *p<0.05 |

| | | |
|---|---|---|
| Statistical significance relative to SAM-P1 control group (Fisher test) | | |

The results shown in Table 6 reveals that the maximal running time of the SAM-P1 control group of the senescence accelerated mice shows a significant decrease compared with that of the SAM-R1 control group of the ordinarily aged mice and that endurance decreases with aging. Moreover, the maximal running time of the mice to which the resveratrol-containing feed was given is significantly long compared with that of the SAM-P1 control group and resveratrol is effective for inhibiting aging-associated reduction in endurance and also effective for improving a motor function.

The results shown in Table 7 reveals that the oxygen consumption of the SAM-P1 control group of the senescence accelerated mice shows a significant decrease compared with that of the SAM-R1 control group of the ordinarily aged mice, and that energy metabolism decreases with aging. Moreover, the oxygen consumption of the mice to which the resveratrol-containing feed was given is significantly high compared with that of the SAM-P1 control group and resveratrol has an inhibitory action against aging-associated reduction in energy metabolism.

The results shown in Table 8 reveals that the percentage of the muscle weight in the body of the SAM-P1 control group of the senescence accelerated mice shows a significant decrease compared with that of the SAM-R1 control group of the ordinarily aged mice and that muscle atrophy occurs with aging. Moreover, the muscle weight of the mice to which the resveratrol-containing feed was given is significantly large compared with that of the SAM-P1 control group.

From the above-described findings, the resveratrol of the present invention is useful as an anti-aging agent, more specifically, an agent for inhibiting aging-associated reduction in endurance, muscle atrophy and reduction in energy metabolism.

### Test 4: Effect of resveratrol for inhibiting reduction in muscle strength

The effect of resveratrol for inhibiting aging-associated reduction in muscle strength was evaluated in the below-described manner. The resveratrol used here is the product of Organic Herb Inc.

After the preliminary individual rearing of 13-week-old SAM-P1 male mice (senescence accelerated mice) and SAM-R1 male mice (ordinarily aged mice) for 5 weeks, they were habituated to treadmill running. Then, they were divided into SAM-R1 control group, SAM-P1 control group, SAM-P1 exercise group, SAM-P1 resveratrol group/exercise (each group consisting of 5 mice). They were reared for 13 weeks on a diet prepared in accordance with the formulation shown in Table 9. Exercise at 15 m/min was imposed to each mouse of the exercise group for 30 minutes three times a week during the period of experiment.

**Table 9**

| Feed composition (wt.%) | | |
|---|---|---|
| | Control group | Resveratrol group |
| Casein | 20% | 20% |
| DL-Methionine | 0.2% | 0.2% |
| Lipid | 10% | 10% |
| Potato starch | 55.5% | 55.3% |
| Cellulose | 8.1% | 8.1% |
| Mineral' | 4% | 4% |
| Vitamin | 2.2% | 2.2% |
| Resveratrol | 0% | 0.2% |
| Total | 100% | 100% |

After rearing for 13 weeks, the mice were sacrificed for dissection. Their extensor digitorum longus musclewas fixed to a transducer ("FORT100", product of World Precision Instruments, Inc.) in a Krebs solution (aeration condition: 95% oxygen, 5% carbon dioxide) of 37°C and then, electrically stimulated (at 140 Hz for 0.5 msec), whereby the maximum muscle strength was measured. The measurement results of the muscle strength are shown in Table 10.

**Table 10**

| Maximum muscle strength of extensor digitorum longus muscle of mice reared for 10 weeks | |
|---|---|
| | Maximum muscle strength (g) |
| SAM-R1 control group | 13.5 ± 1.1 * |
| SAM-P1 control group | 9.8 ± 0.3 |
| SAM-P1 exercise group | 13.0 ± 1.8 |
| SAM-P1 resveratrol/exercise group | 15.0 ± 1.8 * |

| | |
|---|---|
| Statistical significance relative to SAM-P1 control group (Dunnet test) | |

The results shown in Table 10 reveals that the maximum strength of extensor digitorum longus muscle of the SAM-P1 control group which are senescence accelerated mice is significantly low compared with that of the SAM-R1 control group of the ordinarily aged mice and that the maximum muscle strength decreases with aging. Moreover, compared with the SAM-P1 control group, the maximum muscle strength of the mice to which the resveratrol-containing feed was given is significantly high and resveratrol is effective for inhibiting aging-associated reduction in muscle strength.

Based on the above-described finding, the resveratrol of the present invention is useful as an anti-aging agent, more specifically, as an agent for inhibiting aging-associated muscle strength reduction.

### Preparation Examples

Endurance improving, anti-fatigue, muscle strength improving, motor function improving, energy metabolism activating, muscle strength improving, and anti-aging agents (1) to (14) were prepared.

### (1) Fruit juice beverage

A fruit juice beverage having the below-described formulation was prepared.

**Table 11**

| | |
|---|---|
| Resveratrol | 5 mg |
| Vitamin C | 300 mg |
| Grape juice | 300 mL |
| Water | 200 mL |
| Flavor | Certain amount |
| Glucose | 2 mg |

### (2) Capsule

A capsule was prepared by filling the composition (300 mg) shown in Table 12 in a capsule,

**Table 12**

| | | |
|---|---|---|
| Resveratrol | 15 | mass% |
| Vitamin C | 20 | |
| Cellulose | 1.0 | |
| Corn starch | 40 | |
| Tocopherol | 2 | |
| Lactose | 13 | |

### (3) Tablets

Tablets were prepared by tableting the composition (250 mg/tablet) shown in Table 13.

**Table 13**

| | |
|---|---|
| Resveratrol | 10 mass% |
| Corn starch | 50 |
| Cellulose | 10 |
| Lactose | 30 |

### (4) Granules

Granules were prepared by mixing the composition (500 mg/bag) shown in Table 14.

**Table 14**

| | | |
|---|---|---|
| Resveratrol | 5 | mass% |
| Dry grape powder | 20 | |
| Fructose | 30 | |
| Glucose | 25 | |
| Powdered skim milk | 10 | |
| Caffeine | 10 | |

### (5) Food

A food in the form of chewable tablets was prepared by tableting the composition (1000 mg/tablet) shown in Table.15.

**Table 15**

| | | |
|---|---|---|
| Resveratrol | 1 | mass% |
| Lactose | 15 | |
| Maltose | 15 | |
| Glucose | 20 | |
| Glutamine | 10 | |
| vitamin C | 15 | |
| Cellulose | 10 | |
| Caffeine | 4 | |
| Xylitol | 8 | |
| Vitamin E | 1 | |
| Flavor | 1 | |

### (6) Beverage

A beverage was prepared by mixing the composition shown in Table 16.

**Table 16**

| | |
|---|---|
| Water | 500 mL |
| Resveratrol | 10 mg |
| Fructose | 3 g |
| Glucose | 2 g |
| Vitamin C | 500 mg |
| Sodium citrate | 2 g |
| Malic acid | 100 mg |
| Caffeine | 50 mg |
| Flavor | Certain amount |

### (7) Capsule

A capsule was prepared by filling the composition (300 mg) shown in Table 17 in a capsule.

**Table 17**

| | | |
|---|---|---|
| Japanese knotweed extract | 10 | mass% |
| Vitamin C | 20 | |
| Cellulose | 10 | |
| Corn starch | 45 | |
| Tocopherol | 2 | |
| Lactose | 13 | |

### (8) Jelly

The composition shown in Table 18 was dissolved at 65°C and kept at 85°C for 5 minutes. After sterilization, the resulting solution was dispensed into 100-ml containers and allowed to stand for 8 hours. The solution was then gradually cooled to 5°C, whereby a jelly containing resveratrol according to the present invention was prepared.

**Table 18**

| Composition of jelly | (%) |
|---|---|
| Gelling agent mixture (carrageenan and locust bean gum) | 0.65 |
| 50%-concentrated grape juice | 5.00 |
| Citric acid | 0.05 |
| Vitamin C | 0.05 |
| Resveratrol | 0.10 |
| Purified water | Balance |

### (9) Anti-aging enteral nutrient

An anti-aging enteral nutrient of the present invention was prepared by packing the composition shown in Table 19 in a retort pouch in a manner known *per se* in the art and then sterilizing it. As minerals, an organic or inorganic salt mixture of two or more of Na, K, Ca, Mg, P, Cl, Fe and the like was used, while as vitamins, a mixture of two or more of Vitamins A, D, E, B1, B2, B6, B12 and C, niacin, pantothenic acid and the like was used. They were added in an amount satisfying the Japanese Nutritional Requirement.

**Table 19**

| Composition of enteric nutrient | (g/100 mL) |
|---|---|
| Milk casein | 3.40 |
| Isolated soybean protein | 1.67 |
| Dextrin | 14.86 |
| Sucrose | 1.30 |
| Soybean oil | 1.75 |
| Perilla oil | 0.18 |
| Soybean phospholipid | 0.14 |
| Glycerin fatty acid ester | 0.07 |
| Minerals | 0.60 |
| vitamins | 0.06 |
| Resveratrol | 0.005 |
| Purified water | Balance |

### (10) Fruit juice beverage

A fruit juice beverage was prepared in accordance with the formulation shown in Table 20.

**Table 20**

| | |
|---|---|
| Red grape leaf extract | 100 mg |
| Vitamin C | 300 mg |
| Grape juice | 300 mL |
| Water | 200 mL |
| Flavor | Certain amount |
| Glucose | 2 g |

### (11) Capsule

A capsule was prepared by filling the composition (300 mg) shown in Table 21 in a capsule.

**Table 21**

| | |
|---|---|
| Red grape leaf extract | 25 mass% |
| Vitamin C | 20 |
| Cellulose | 10 |
| Corn starch | 30 |
| Tocopherol | 2 |
| Lactose | 13 |

### (12) Tablets

Tablets were prepared by tableting the composition (250 mg/tablet) shown in Table 22.

**Table 22**

| | |
|---|---|
| Red grape leaf extract | 25 mass% |
| Corn starch | 35 |
| Cellulose | 10 |
| Lactose | 30 |

### (13) Food

A food in the form of chewable tablets was prepared by tableting the composition (1000 mg/tablet) shown in Table 23.

**Table 23**

| | |
|---|---|
| Red grape leaf extract | 3 mass% |
| Lactose | 13 |
| Maltose | 15 |
| Glucose | 20 |
| Glutamine | 10 |
| Vitamin C | 15 |
| Cellulose | 10 |
| Caffeine | 4 |
| Xylitol | 8 |
| Vitamin E | 1 |
| Flavor | 1 |

### (14) Beverage

A beverage was prepared by mixing the composition shown in Table 24.

**Table 24**

| | |
|---|---|
| Water | 500 mL |
| Red grape leaf extract | 50 mg |
| Resveratrol | 20 mg |
| Fructose | 3 g |
| Glucose | 2 g |
| Vitamin C | 500 mg |
| Sodium citrate | 2 g |
| Malic acid | 100 mg |
| Caffeine | 50 mg |
| Flavor | Certain amount |

### (15) Beverage

A sports drink of the present invention was prepared in accordance with the formulation and conditions shown in Table 25. The beverage thus obtained had good storage stability and provided good taste.

**Table 25**

| (g/100 mL) | | | | |
|---|---|---|---|---|
| | Formulation example 1 | Formulation example 2 | Formulation example 3 | Formulation example 4 |
| Resveratrol (g) | 0.01 | 0 | 0.02 | 0.008 |
| Red grape leaf extract (g) | 0 | 0.04 | 0 | 0.01 |
| Sweetener (Aspartame + Glucose (g)) | 1.2 | 1.2 | 1.5 | 1.5 |
| Acidifier (g) | 0.24 | 0.24 | 0.24 | 0.24 |
| Purified water (g) | Balance | Balance | Balance | Balance |
| NaCl (g) | 0.04 | 0,04 | 0.04 | 0.04 |
| KCl (g) | 0.009 | 0.009 | 0.009 | 0.009 |
| Flavor (g) | 0,3 | 0.3 | 0.3 | 0.3 |
| Vitamin C (g) | 0.03 | 0,03 | 0.1 | 0.05 |
| pH (after sterilization) | 3.5 | 3.5 | 3.5 | 3.5 |
| Container | PET container | Aluminum container | PET container | Aluminum container |

## Claims

1. Non-therapeutic use of resveratrol for improving aging-induced reduction of energy metabolism in a human or animal.

2. Non-therapeutic use of resveratrol for improving endurance in exercise in a human or animal.

3. Non-therapeutic use of resveratrol for anti-fatigue in exercise in a human or animal.

## Patentansprüche

1. Nichttherapeutische Verwendung von Resveratrol zur Verbesserung der altersinduzierten Reduktion des Energiestoffwechsels bei einem Menschen oder Tier.

2. Nichttherapeutische Verwendung von Resveratrol zur Verbesserung der Ausdauer bei Betätigung bei einem Menschen oder Tier.

3. Nichttherapeutische Verwendung von Resveratrol gegen Ermüdung bei Betätigung bei einem Menschen oder Tier.

## Revendications

1. Utilisation non thérapeutique de resvératrol pour améliorer la réduction du métabolisme énergétique induite par le vieillissement chez un être humain ou un animal.

2. Utilisation non thérapeutique de resvératrol pour améliorer l'endurance durant l'exercice chez un être humain ou un animal.

3. Utilisation non thérapeutique de resvératrol pour lutter contre la fatigue durant l'exercice chez un être humain ou un animal.
